# EUROPEAN PATENT SPECIFICATION

(11) **EP 2 185 167 B1**
(45) Date of publication and mention of the grant of the patent: **04.07.2018**
(21) Application number: 08830472.0
(22) Date of filing: 12.09.2008
(51) Int. Cl.: A61K 36/00, G01N 30/86, G01N 33/15, G01N 1/28

(54) **SYSTEM AND METHOD FOR ASSESSING TRADITIONAL MEDICINES**
SYSTEM UND VERFAHREN ZUM TESTEN TRADITIONELLER MEDIKAMENTE
SYSTEME ET PROCEDE D'EVALUATION DES MEDICAMENTS TRADITIONNELS

(30) Priority: 14.09.2007 US 972651 P
(43) Date of publication of application: 19.05.2010
(73) Proprietor: PHYTO CZ, s.r.o., 79601 Prostejov (CZ); Pennyroyal, Greg A., Temecula, CA 92590 (US)
(72) Inventor: LEUNG, Albert Y., Glen Rock, NJ 07452-2603 (US); PENNYROYAL, Greg A., Temecula, CA 92590 (US)
(74) Representative: Dvorakova, Martina
(86) International application number: PCT/US2008/076310
(87) International publication number: WO 2009/036387

(56) References cited:
- WO-A-02/058625
- US-A1- 2003 152 651
- SPRINGFIELD E P ET AL: "Quality assessment of South African herbal medicines by means of HPLC fingerprinting" JOURNAL OF ETHNOPHARMACOLOGY, ELSEVIER SCIENTIFIC PUBLISHERS LTD, IE, vol. 101, no. 1-3, 3 October 2005 (2005-10-03), pages 75-83, XP025270003 ISSN: 0378-8741 [retrieved on 2005-10-03]
- CHEN ET AL: "Capillary high-performance liquid chromatography with mass spectrometry for simultaneous determination of major flavonoids, iridoid glucosides and saponins in Flos Lonicerae" JOURNAL OF CHROMATOGRAPHY, ELSEVIER SCIENCE PUBLISHERS B.V. AMSTERDAM, NL, vol. 1157, no. 1-2, 26 June 2007 (2007-06-26), pages 217-226, XP022131714 ISSN: 0021-9673
- TSAI T-R ET AL: "Identification and determination of geniposide contained in Gardenia jasminoides and in two preparations of mixed traditional Chinese medicines" JOURNAL OF CHROMATOGRAPHY, ELSEVIER SCIENCE PUBLISHERS B.V. AMSTERDAM, NL, vol. 961, no. 1, 28 June 2002 (2002-06-28), pages 83-88, XP004369774 ISSN: 0021-9673
- ZHANG ET AL: "A gradient HPLC method for the quality control of chlorogenic acid, linarin and luteolin in Flos Chrysanthemi Indici suppository" JOURNAL OF PHARMACEUTICAL AND BIOMEDICAL ANALYSIS, NEW YORK, NY, US, vol. 43, no. 2, 23 December 2006 (2006-12-23), pages 753-757, XP005735150 ISSN: 0731-7085

## Description

### CROSS-REFERENCE TO RELATED APPLICATIONS

This application claims priority to U.S. Provisional Application No. 60/972,651, entitled "METHOD FOR QUALITY CONTROL AND ACCESSING EFFICACY OF NATURAL THERAPEUTIC MATERIALS," filed September 14, 2007, which is herein incorporated by reference.

### STATEMENT REGARDING FEDERALLY SPONSORED R&D

Certain aspects of the invention disclosed herein were made with United States government support under Department of Health and Human Services Grant No. 5R44AT000770-03. The government has certain rights in these aspects of the invention.

### BACKGROUND

### Technical Field

This disclosure relates to systems and methods for assessing traditional Chinese medicines and more particularly to scientifically-reproducible systems and methods useful for quality assurance (QA) and quality control (QC) of traditional Chinese medicines.

### Description of the Related Art

Traditional medicines are plant-, animal-, and mineral-based foods, cosmetics, and therapeutics that are used to treat or prevent illness or maintain well-being. The most common and often the most complex traditional medicines are plant-based therapeutics such as herbs.

Evidence documenting the use of traditional medicines appears in Chinese literature dating back more than 4,000 years. Since then, traditional medicines have been extensively used in Asia and around the world as a primary form of treatment. The use of traditional medicines has recently experienced a resurgence in Western countries and become part of a multibillion-dollar herbal industry. Accordingly, Western scientists have begun to explore the biomedical and pharmaceutical potential of traditional medicines.

The traditional QA/QC practices of experts in traditional medicine are based upon carefully documented empirical observations made over time. These observations are based upon the qualities, physical characteristics, and therapeutic functions of traditional medicines from a variety of sources, possible substitutions for the medicines, as well as particular combinations of medicines which when combined, are described as having particular synergistic therapeutic effects. The observations are documented in traditional medicine pharmacopeia and/or materia medica, the body of collected knowledge about the therapeutic properties of any substance used for healing.

Traditional QA/QC practices do not meet Western scientific requirements of reproducibility, verification, and quantification. Thus, Western scientists have applied their knowledge of characterizing modern allopathic drugs in an attempt to develop scientifically-reproducible methods for assessing traditional medicines. The work typically focuses on the analysis, isolation, and identification of chemical compounds in single herbs and characterizing their pharmacological properties.

However, Western research on the efficacy and safety of traditional medicines is deficient for a variety of reasons. One important drawback is that the studies have ignored the medicines' traditional theories, properties, and indications.

Previous Western research emphasizes the effects of isolated active ingredients of single herbs. Allopathic drugs typically comprise a single, high-purity active ingredient that is easy to identify and quantify using existing standard analytical techniques. Traditional medicines are very different from allopathic drugs. Because they are plant-, animal-, and mineral-derived natural products, traditional medicines are almost always complex formulations containing many different chemical components. Even within a single herb, numerous groups of components work in various complementary, supporting, antagonistic, and/or synergistic ways. Moreover, use of single herbs does not reflect multi- herb traditional formulas and ignores traditional theories of herb interactions. Many remedies according to traditional practice are themselves mixtures of these complex formulations.

The previous research also fails to account for traditional medicines from diverse sources. Indigenous regions originally supplied the traditional medicines. Documentation of the medicines' properties, uses, and efficacy is largely based on observations based upon traditional medicines from indigenous regions or other traditional sources. Traditional medicines harvested from non- indigenous regions or other non- traditional sources do not necessarily have the same therapeutic properties as their taxonomically-identical counterparts from indigenous regions or other traditional sources.

Patent Applications WO02/058625 and US2003/152651 describe how to manufacture standardized DSP (Dan Shen Pill) medicaments utilizing the purified standards relevant to the specific therapeutic substances in the medicament. The standards are presented as purified 'single' substance of the manufactured medicine or composition of solutions containing purified relevant substances. Subsequently the analytical profile of these standards are provided.

Scientific Article "Quality assessment of South African herbal medicines by means of HPLC fingerprinting" by Springfield et al., vol.101, no. 1-3, 2005, describes method of controlling the quality of South African plant species using HPLC-DAD "fingerprints". The disadvantage of this method is that standards for plant species are established without qualitative profiling. Plant are gathered in its natural habitant at different season, from different geographical locations and its different soil types that affect the therapeutic properties of such "standards".

Accordingly, purification and isolation of specific "active" compounds do not accurately reflect the quality and efficacy of a traditional medicine. A need remains for scientifically-reproducible systems and methods for assessing traditional medicines that overcome these deficiencies.

### SUMMARY

An aspect of at least one of the embodiments disclosed herein includes the realization that scientifically-reproducible systems and methods for assessing traditional Chinese medicines can be implemented that do not require the isolation and/or identification of active constituents. The broad spectrum of components in a traditional Chinese medicine can be characterized without inquiring into the components' identities. In certain embodiments, traditional, empirical methods of assessing traditional Chinese medicine can be captured in a scientifically-reproducible manner that complies with Western scientific requirements of reproducibility, verification, and quantification.

In one embodiments, a method of defining a standard reference sample for a traditional Chinese medicine is provided. The method comprising:
a) obtaining at least two speciments of the whole traditional Chinese medicine consisting of *Panax quinquefolius, Panax ginseng, Curcuma spp., Ganoderma lucidum, Ganoderma sinense* that have been authenticated as representing a positive control by qualitative profiling using an organoleptic examination;
b) quantitatively profiling each of the at least two specimens that are authenticated as the positive control in step a) using High Performance Thin Layer Chromatography in combination of High-Performance Liquid Chromatography, Fourier Transform Infrared Spectroscopy and/or Gas-Liquid Chromatography and quantitatively profiling at least one of the at least two speciments using at least one biologic/genomic analysis selected from group consisting of proteomic assay, clinical trial, and DNA microarray to confirm positive authenticity of the specimens;
c) blending at least two speciments that are authenticated as the positive control in step a) and confirmed in the step b) to form the standard reference sample;
d) and creating a quantitative profile for the standard reference sample using High Performance Thin Layer Chromatography in combination of High-Performance Liquid Chromatography, Fourier Transform Infrared Spectroscopy and/or Gas-Liquid Chromatography and quantitatively profiling of the standard reference sample using at least one biologic/genomic analysis selected from group consisting of proteomic assay, clinical trial, and DNA microarray; wherein the quantitative profile for the standard reference sample defines the standard reference sample.

In another embodiment, a method of evaluating a test specimen of a whole traditional Chinese medicine is provided. The method comprising:
a) creating a quantitative profile for the test specimen using at least two physicochemical analyses selected from the group consisting of Mass Spectrometry, High Performance Thin Layer Chromatography, Fourier Transform Infrared Spectroscopy, Ultraviolet- Visible Spectroscopy, Thin Layer Chromatography, Gas-Liquid Chromatography, High-Performance Liquid Chromatography, Liquid Chromatography-Mass Spectrometry, and Gas Chromatography-Mass Spectrometry and creating the quantitative profile for the test specimen using at least one biologic/genomic analysis selected from the group consisting of proteomic assay, clinical trial and DNA microarray or combination thereof;
b) providing a standard reference sample defined by the method according to the invention;
c) comparing the quantitative profile for the standard reference sample to the quantitative profile for the test specimen mathematicaly or visually;
d) evaluation being reached when the variation between the test specimen and the standard reference sample is 25% or less, preferably 10% or less or the most preferably below 5%.

In certain embodiments, the method of evaluating a test specimen further comprises authenticating the test specimen by qualitative profiling.

In another embodiment, an evaluated traditional Chinese medicine is provided comprising a traditional medicine evaluated by the methods described above.

For purposes of summarizing the embodiments and the advantages achieved over the prior art, certain items and advantages are described herein. Of course, it is to be understood that not necessarily all such items or advantages may be achieved in accordance with any particular embodiment. Thus, for example, those skilled in the art will recognize that the inventions may be embodied or carried out in a manner that achieves or optimizes one advantage or group of advantages as taught or suggested herein without necessarily achieving other advantages as may be taught or suggested herein. The flow charts described herein do not imply a fixed order to the steps, and embodiments of the invention may be practiced in any order that is practicable. Although the primary application for this technology is for traditional Chinese medicine, the systems and methods disclosed herein can be used to identify, characterize, and standardize any natural material irrespective of its traditional use or lack thereof as long as initial specimens to be analyzed and matched are available.

### BRIEF DESCRIPTION OF THE DRAWINGS

A general architecture that implements the various features of the disclosed systems and methods will be described with reference to the drawings. The drawings and the associated descriptions are provided to illustrate embodiments and not to limit the scope of the disclosure. Throughout the drawings, reference numbers are re-used to indicate correspondence between referenced elements. In addition, the first digit of each reference number indicates the figure in which the element first appears.
FIG. 1 is a block diagram showing an overview of the problem with assessing traditional Chinese medicine.
FIG. 2A is a block diagram illustrating a method of defining a standard reference sample for a traditional Chinese medicine according to one embodiment. FIG. 2B is a block diagram illustrating a method of evaluating a test specimen of a traditional Chinese medicine according to one embodiment.
FIG. 3A is an example botanical taxonomical key created from macroscopic analysis. FIG. 3B is an example comparative matrix created from macroscopic analysis.
FIG. 4 shows Gas Chromatograph profiles of three traditional Chinese medicine specimens.
FIG. 5 shows High Performance Thin Layer Chromatography profiles of three traditional Chinese medicine specimens.
FIG. 6A and FIG. 6B show High Performance Liquid Chromatography profiles for two traditional Chinese medicine specimens.
FIG. 7A and FIG. 7B show High Performance Liquid Chromatography profiles for two traditional Chinese medicine specimens.
FIG. 8A and FIG. 8B show High Performance Liquid Chromatography profiles for two traditional Chinese medicine specimens.
FIG. 9 shows Fourier Transform Infrared Spectroscopy profiles for three traditional Chinese medicine specimens.
FIG. 10 shows UV-Visible Spectroscopy profiles of three traditional Chinese medicine specimens.

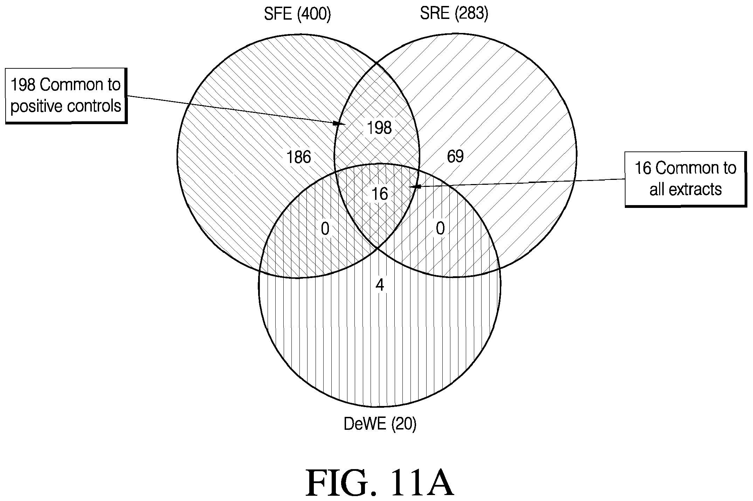

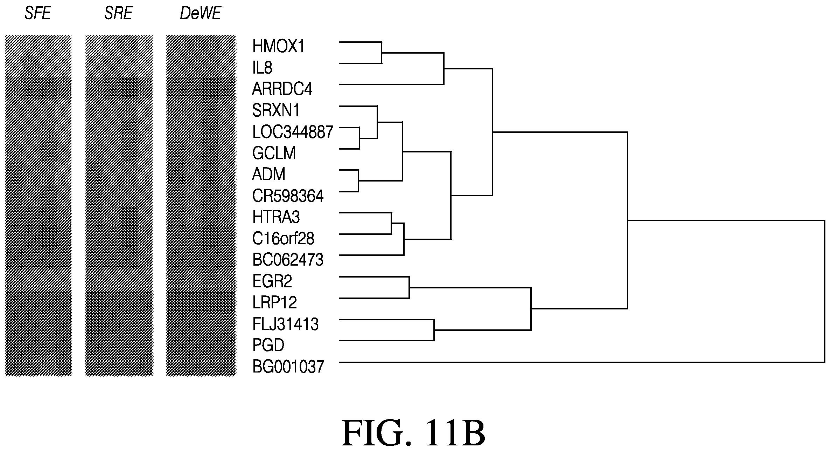

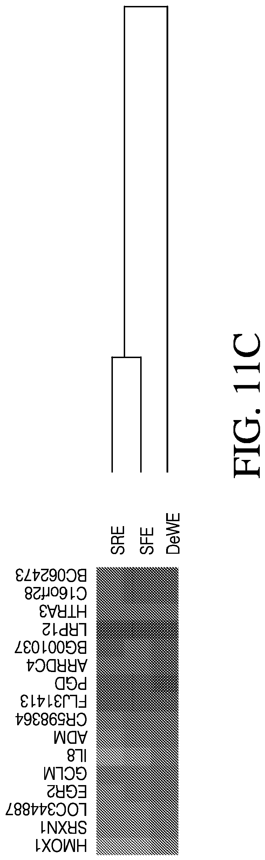

FIG. 11A is a Venn diagram showing numbers of significant gene responses in a gene microarray to three traditional Chinese medicine specimens. FIG. 11B and FIG. 11C show related expression profiles.
FIG. 12 shows gene response patterns to traditional Chinese medicine specimens.
FIG. 13A is table containing source information on specimens comprising American and Asian Ginseng. FIG. 13B shows UV-Visible Spectra for specimens of American and Asian Ginseng.
FIG. 14 depicts physical differences between turmeric, ezhu, and yujin specimens.
FIG. 15A depicts UV-Visible Spectroscopy profiles of turmeric, ezhu, and yujin specimens. FIG. 15B depicts HPTLC profiles of turmeric, ezhu, and yujin specimens.
FIG. 16 depicts physical differences between ganoderma specimens.
FIG. 17A depicts UV-Visible Spectroscopy profiles of ganoderma specimens. FIG. 17B depicts HPTLC profiles of ganoderma specimens.
FIG. 18 is a Venn diagram showing numbers of significant gene responses in a DNA microarray to ganoderma specimens

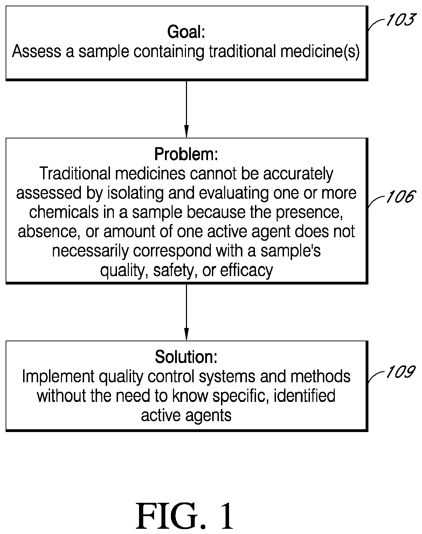

Traditional medicines are plant-, animal-, and mineral-based foods, cosmetics, or therapeutics that are used to treat or prevent illness or maintain well-being. Examples of traditional medicines include Traditional Chinese Medicines and Ayurvedic Medicines. For a more detailed understanding of the disclosure, reference is first made to FIG. 1, which illustrates an overview of the problem of assessing traditional Chinese medicines. As explained in block 103, a user seeks to assess a speciemen containing a traditional Chinese medicine.

However, as explained in block 106, the active agents or the therapeutic combination of agents in the specimen are often unknown or undetectable. Moreover, the presence, absence, or amount of one active agent in a traditional Chinese medicine does not necessarily correspond with a specimen's quality, safety, or efficacy. Consequently, traditional Chinese medicines cannot be accurately assessed by isolating and evaluating one or more chemicals in a specimen.

Therefore, as described in block 109, systems and methods are provided to assess a traditional Chinese medicine specimen's quality, safety, efficacy, etc. without the need to isolate and/or identify active agents in the specimen. This approach takes account of the broad spectrum of components in a traditional medicine using both qualitative and quantitative profiling.

An example system of characterizing a traditional Chinese medicine comprises one or more qualitative profiling techniques, one or more quantitative profiling techniques, and data storage.

### Qualitative Profiling

The one or more qualitative profiling techniques create an initial link to tradition. These profiling techniques reflect the traditional QA/QC practices of experts in traditional Chinese medicine. The one or more qualitative profiling techniques can include, but are not limited to, profiling by one or more experts in traditional Chinese medicine practice and quality and/or research of traditional or modern documentation. The information obtained from the experts and/or documentation can be stored and indexed in a computer database.

### 1.Subjective Evaluation

An expert in Traditional Chinese Medicine, can be consulted to profile and authenticate a specimen of a traditional Chinese medicine. For example, an expert can authenticate the quality of a specimen using traditional quality control methods such as organoleptic examination and/or other traditional process controls. Organoleptic examination comprises sensory observations of the general features, size, texture, surface characters, color, fracture features, flavor, taste, etc. of a specimen. The subjective evaluation of one or more experts using traditional quality control methods creates a strong link to systematic traditional Chinese medicine.

### 2. Objective Evaluation

Research of traditional and/or modern documentation can comprise, among other things, obtaining information such as traditional names(s), efficacy in clinical trials, time of harvest, region of harvest, plant part, plant maturity, cultivation, harvest and handling specifications, post-harvest processing, taxonomy, toxicity, known and potential adulterants, quality assurance and quality control issues, and regional differences, etc.

The one or more qualitative profiling techniques can authenticate a specimen as a "control positive," that is a specimen that represents the quality of traditional Chinese medicine as documented in traditional literature and/or practice. The one or more qualitative profiling techniques can also authenticate a specimen as a "control negative, " that is a specimen that does not represent the quality of material as documented in traditional literature and/or practice because of poor quality, known adulterants, or origination from a non-indigenous region or other non-traditional source, etc.

Expert consultation and research of traditional or modern documentation can be used individually or in combination. For example, a specimen can be selected which conforms with the time of harvest, plant part, plant maturity, and handling specifications as described in the traditional literature. An expert can subsequently perform an organoleptic examination of the specimens to verify that it represents the quality of material as documented in traditional literature and/or practice.

### Quantitative Profiling

The one or more quantitative profiling techniques can comprise a plurality of physicochemical characterizations or, preferably, a combination of physicochemical characterizations and biologic/genomic analysis. The data from one or more of the quantitative profiling techniques can be stored and indexed in a computer database.

As discussed above, traditional Chinese medicines are inherently complex substances. The overall efficacy of traditional Chinese medicines is not solely derived from a single active component but rather from multiple active components and/or auxiliary components of these medicines. Furthermore, like traditional Chinese medicines, biological systems are inherently complex, and bioactive compounds in traditional Chinese medicines have complex interactions with biological systems. The physicochemical and biologic/genomic characterizations are consequently selected to capture and quantify the broad spectrum of components present in a traditional Chinese medicine specimen without demanding any inquiry into the identity of the components themselves or their specific bio activity pathways.

One or more quantitative profiling techniques are performed upon a control positive or a control negative as described above. The specimen is a blend of control positives. Blending of control positives advantageously creates a standard reference sample that includes a range of variation inherent in the traditional Chinese medicine as it has been traditionally used and documented.

### 1. Physicochemical Fingerprint

Physicochemical characterizations are selected to capture and quantify a broad and comprehensive physical and chemical profile (physicochemical fingerprint) for a specimen. At least two physicochemical characterization tools are used to create a physicochemical fingerprint. Suitable physicochemical characterization tools include, but are not limited to, microscopy, macroscopy, mass spectrometry, spectroscopy such as Fourier Transform Infrared (FTIR) Spectroscopy and Ultraviolet- Visible (UV/V1S) Spectroscopy, chromatography such as Thin Layer Chromatography (TLC) and High Performance Thin Layer Chromatography (HPTLC), Gas-Liquid Chromatography (GLC), and High- Performance Liquid Chromatography (HPLC), and combination characterizations such as Gas Chromatography-Mass Spectrometry (GC-MS) and Liquid Chromatography-Mass Spectrometry (LC-MS).

HPTLC is used in conjunction with a plurality of other physicochemical characterizations. For example, over multiple solvent systems (e.g. polar and non-polar), HPTLC can be combined with a specific test such as HPLC and differing analytical tools such as spectroscopy (FTIR) and/or chromatographic separation (e.g. GLC) and other chromatographic techniques.

In contrast to Western research which seeks to characterize traditional Chinese medicine specimens by isolating and identifying active ingredients of single herbs, the systems and methods disclosed herein develop a comprehensive physicochemical fingerprint. Physicochemical fingerprinting does not require an investigation into the individual components that make up a traditional Chinese medicine. Indeed, the purportedly "active" ingredients are not isolated from a traditional Chinese medicine or remedy comprising several traditional Chinese medicines prior to physicochemical characterization. Instead, the quality and efficacy of a traditional Chinese medicine or remedy specimen can be based upon patterns in the data gathered from a whole specimen using various physicochemical characterization techniques. These data reflect the full spectrum of components in the specimen which respond to the physicochemical characterization techniques.

### 2. Biologic/Genomic Fingerprint

Biologic/genomic analysis can comprise one or more biologic/genomic characterizations selected to capture and quantify a biologic/genomic fingerprint for a specimen.

Suitable biologic/genomic characterizations include, but are not limited to, genetic assays, proteomic assays, biological assays, and clinical trials. Preferably the biologic/genomic characterizations integrate molecular, cellular, and whole organismal information to capture dynamic biochemical processes and complex physiological interactions.

An example biologic/genomic characterization comprises proteomic tandem mass spectrometry, which determines the identity, modification states, and relative abundance of proteins.

The biologic/genomic characterization comprises microarray analysis. An example microarray analysis comprises DNA microarray, which advantageously does not require a priori assumptions or knowledge of the biological system. A DNA microarray is a high-throughput technology comprising an arrayed series of thousands of microscopic spots of DNA features.

DNA microarrays can be used for gene expression profiling and assessing genome content. Microarray technology allows simultaneous measurement of the gene expression of the entire genome and can provide a global view of gene-gene interactions and biological processes. A DNA microarray can also focus on a subset of the genome, specific genes, a portion of a gene, or other DNA elements. Subsets of the entire genome and/or specific genes show unique patterns of response to traditional Chinese medicines. These subsets and/or genes can be selected for a specific known actions such as anti-inflammatory activity or selected for unique and/or strength of signal response or any combination of these.

Smaller subsets of the entire genome and/or specific genes show unique patterns of response to a standard reference sample. One or more of these subsets can be selected for a specific known action(s) such as anti-inflammatory activity or selected for unique and/or strength of signal response or any combination of these. One or more subsets can be used in unique select gene panels that are practical for routine QA/QC testing.

A preferred biologic/genomic characterization comprises a bioassay, which is used to measure the effects of a substance on a living organism. The use of bioassays advantageously enhances reproducibility and quality control and permits insights into biological activity. A variety of bioassays can suitably provide relevant information in the characterization of a traditional Chinese medicine.

In contrast to previous Western research which seeks to characterize traditional Chinese medicine specimen by isolating and identifying active ingredients of single herbs, the systems and methods disclosed herein develop a biologic/genomic fingerprint. Biologic/genomic fingerprinting does not require an investigation into the individual components that make up a traditional Chinese medicine. Indeed, purported "active" ingredients are not isolated from a traditional Chinese medicine or remedy comprising several traditional medicines prior to physicochemical characterization. Instead, the quality and efficacy of a traditional Chinese medicine or remedy specimen can be based upon patterns in the data gathered from a whole specimen. This can allow research into the effects of auxiliary components in a specimen upon the efficacy of the principal component, enhancement of the selectivity or bioavailability of the principal component, and suppression of unwanted side effects. These data reflect the full spectrum of components in the specimen which respond to the biologic/genomic characterization techniques used.

The combination of physicochemical and biologic/genomic characterization can be advantageous. The spectrum of components in a traditional Chinese medicine or remedy can show a clinical effect that may be attributed principally to one component but which is considerably modified by the presence of other components. Biologic/genomic profiling can provide a quantitative measure of the biologic or genomic effects of a traditional Chinese medicine and complement or confirm the information obtained from physicochemical characterization.

Both physicochemical and biologic/genomic characterizations are performed on all specimens. Only physicochemical characterizations are performed on specimens. All specimens are subject to physicochemical characterization and a subset of the specimens are also subject to biologic/genomic characterization. For example, a biologic/genomic characterization can be used to validate and reinforce a decision to accept or reject a test specimen as compliant or non-compliant with a standard reference sample (as described below).

### Defining a Standard reference sample

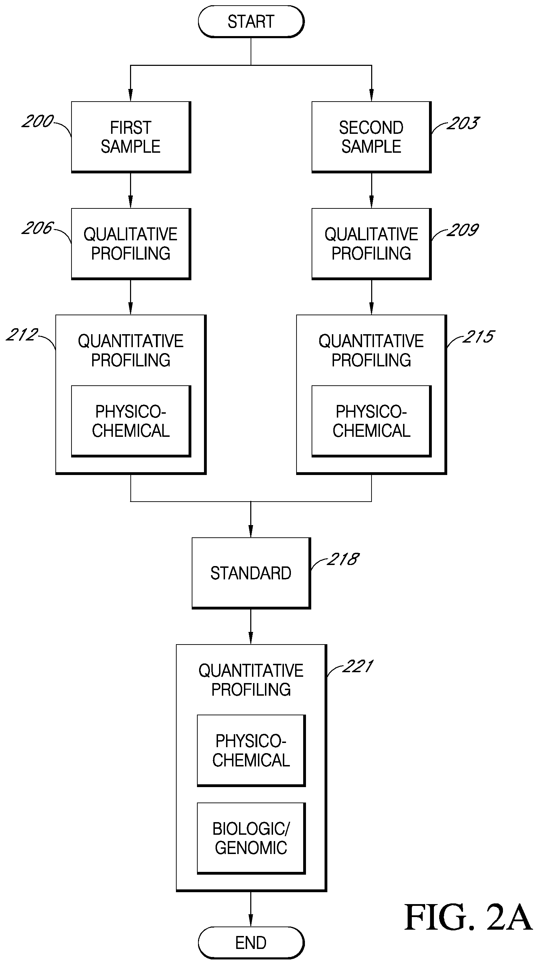

An example is shown in FIG. 2A. A first specimen 200 and a second specimen 203 of a traditional Chinese medicine are obtained from two different traditional growing regions, traditional distribution points, or other traditional sources. In the example two specimens are obtained. More preferably, at least three or at least four specimens are obtained. A greater number of initial specimens advantageously improves the statistical significance or accuracy of subsequent analyses. A greater number of initial specimens can better capture the variation inherently present in the natural medicine as it has been traditionally practiced and documented.

Qualitative profiling 206, 209 is performed upon each specimen 200, 203 to authenticate that each specimen 200, 203 represents the quality of traditional Chinese medicine as documented in traditional literature and/or practice. Qualitative profiling 206, 209 authenticates the first specimen 200 and second specimen 203 as control positive specimen.

After qualitative profiling 206, 209, each specimen is then subject to one or more quantitative profiling techniques 212, 215. For example, the first specimen 200 and the second specimen 203 are each subject to a plurality of physicochemical characterizations. The results of the physicochemical characterizations are analyzed to confirm that neither specimen verifying that neither specimen represents an outlier specimen (for example, a specimen containing adulterants). If a specimen as identified as an outlier specimen, the specimen can be rejected. A new specimen can be obtained and authenticated by qualitative profiling as representing a control positive.

If neither specimen represents an outlier specimen, the first specimen 200 and the second specimen 203 can be blended into a standard reference sample 218. The standard reference sample 218 thus includes a range of variation inherent in the traditional Chinese medicine as it has been traditionally used and documented. Quantitative profiling 221 can be performed upon the standard reference sample 218. The results of the quantitative profiling can be stored and can serve to define the standard reference sample 218 for the traditional Chinese medicine. The definition of the standard reference sample 218 can be later refined by integrating the results of additional quantitative analyses on control positive and/or control negative specimens. For example, additional specimens can be blended into the standard reference sample, which is subsequently subject to additional quantitative profiling. Data from multiple standard reference samples can be gathered and stored. The physicochemical and/or biologic/genomic fingerprints from multiple standard reference samples can be used to statistically derive a definition based upon the range of variation observed across the fingerprints. The above-described techniques work without the need to know the potentially vast number of biologically active components in the traditional Chinese medicine specimens.

The quantitatively profiled control positive specimens are not blended prior to defining a standard reference sample for the traditional Chinese medicine. For instance, the fingerprints from multiple control positive specimens can be used to statistically derive a definition for a standard reference sample based upon the range of variation observed in the fingerprints. The definition for the standard reference sample can be later refined by integrating the results of additional quantitative analyses on control positive and/or control-negative specimens.

### Evaluating Test Specimens

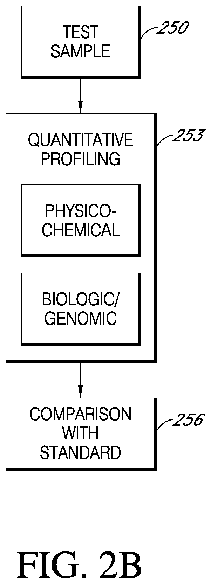

As shown in FIG. 2B, a specimen need not be qualitatively profiled as either a control positive or negative to undergo quantitative analysis. For example, a test specimen has not been qualitatively profiled and represents a specimen of unknown quality 250. The test specimen 250 is quantitatively profiled 253, and the results can be stored in a computer database. The results of the quantitative profile 253 for the test specimen 250 can be compared 256 to the standard reference sample described above, for example, using ïnformatic software on a computer or by visual comparison. If the quantitative profile 253 for the test specimen 250 conforms with the standard reference sample, then the specimen of unknown quality 250 represents the quality of material as documented in traditional literature and/or practice. Such a test specimen can be said to be a evaluated specimen. This process advantageously provides a evaluated specimen with a strong link to tradition and integrates traditional QA/QC practices while meeting Western scientific requirements of reproducibility, verification, and quantification. This process provides a evaluated specimen of a traditional Chinese material that represents the safety and efficacy established by traditional practice and documentation.

Comparison of the test specimen to the standard reference sample is accomplished by visually comparing the at least two physicochemical profiles obtained for the test specimen and the standard reference sample. This visual comparison can include for example, examining the respective HPTLC chromatograms for the test specimen and the standard reference sample for blot pattern similarities and differences. Preferably, comparison of the test specimen to the standard reference sample is accomplished by mathematically, statistically, or otherwise objectively comparing the at least two physicochemical profiles obtained for the test specimen and the standard reference sample, for example using informatic software. This mathematical comparison can include quantification and comparison of the relative blot migration and optical density between the test specimen and the standard reference sample. This can include measuring peak heights and areas under the curve for comparing chromatograms (HPLC, etc).

Evaluation is reached when the variation between the test specimen and the standard reference sample is within 0-25%, and more preferably between about 0-10% and most preferably below about 5%.

A specimen that has been qualitatively profiled as a control negative can be a test specimen which is compared to a standard reference sample or a control positive described above. Valuable insights can be obtained through informatic processing of the differences between the quantitative profiles for the control negative and the standard reference sample or control positive. For example, comparison of a control negative to a control positive can be used to identify gene profiles responsive to a particular traditional Chinese medicine.

The systems and methods disclosed herein have certain advantageous distinctions over U.S. Patent No. 6,806,090 to Hylands et al. ("Hylands"). Hylands is directed to a process for quality control and standardization of medicinal plant products. The process of that invention provides a means of defining a standard reference sample for a given medicinal plant material on the basis of a known specimen of the material which possesses the particular property desired for the standard reference sample. A specification for the standard reference sample is established by submitting the known specimen to (a) a combination of NMR spectroscopy and a computer- based pattern recognition technique and (b) one or more biological profiling techniques, and defining the results thus obtained as the standard reference sample specification. Subsequent "candidate" specimen of the plant material can then be tested for compliance with the standard reference sample.

As described in more detail below, in contrast to systems and methods described herein, Hylands does not capture traditional systems of medicine within a standardized system of inquiry and data collection. Furthermore, Hylands does not capture the naturally occurring variation of traditionally-proven therapeutics.

Hylands uses a single sample of a plant or plant-based therapeutic material that purportedly possesses the "property desired for the standard". Hylands assumes that the single sample that possesses the property desired is representative of all material used as the traditional medicine. This assumption is flawed. For example, Applicants have shown samples collected from the same plant species exhibit different physicochemical profiles based upon, for example, time of harvest. The Hylands model does not account for this natural variation.

Basing the property desired for the standard on the qualities of a single sample fails to consider the range of suitable therapeutic plant materials, processes, and clinical application that have been defined as the traditional medicine by traditional practitioners through careful observation, documentation, and adherence to a theory of medicine. The standard referred to in Hylands does not reflect the material used in traditional medicine as documented in traditional literature and/or practice. Rather, the "standard" simply reflects a single sample that has been shown to possess some presumed property.

It is disclosed herein the realization that a control positive to which subsequent specimens are compared should be authenticated through qualitatively profiling as representing the quality of traditional Chinese medicine as documented in traditional literature and/or practice.

Hylands assumes that there is presently at best a crude system of characterization, control, and quality consistency monographs in traditional Chinese medicine. For example, Hylands states that it is virtually impossible to provide any assurance that specimens of a given plant material obtained from disparate sources will possess a uniform identity and biological activity.

However, many traditional systems of medicine, such as traditional Chinese medicine, have rigorous systems of process standardization. For example, the traditional Chinese materia medica contains highly detailed monographs for all stages of production and process control. The materia medica provides a centralized and standardized system of reporting prior findings and integrating new findings developed over the last three millennia.

Thus, in sharp contrast to Hylands, it is disclosed herein the realization that by qualitatively profiling a traditional Chinese medicine, it is possible to identify a range of composition that the tradition identifies as representing the quality of traditional Chinese medicine as documented in traditional literature and/or practice. Through qualitative profiling, this composition can be distinguished from materials traditionally identified as inferior or from adulterated materials not following the careful processes set forth in traditional practice and documented in the traditional literature.

Hylands uses only NMR spectroscopy and pattern recognition analysis for analyzing chemical compositions. NMR Spectroscopy can be used to identify detailed specific composition and structures of molecules, especially in complex chemical matrices. The use of NMR Spectroscopy is consistent with Hylands' s goal of defining the specific chemical composition of an herbal formula as a method of identification and consistency.

However, NMR Spectroscopy alone fails to capture the total range of natural variation that is defined as a traditional Chinese medicine. Plants that are grown, harvested, and processed in a specific manner specifying climate, region, ecological variations, time of harvest, etc. will express chemically slightly differently as individuals and with some variation within a traditional farming and collection region, even using standardized practices.

I tis realized that the combined physicochemical composition of an overall population group should be characterized to capture the total range of natural variation that is defined as a traditional Chinese medicine. The systems and methods disclosed herein accordingly comprise a wider series of physicochemical analytical techniques.

As explained above, data obtained from the one or more qualitative profiling techniques and/or the quantitative profiling techniques can be indexed and stored in data storage, for example computer database(s). Data stored in the database(s) can be used for data mining and feedback loops. For example, as future research is done, additional variables can be tested and compared using physicochemical and biologic/genomic analysis to address such questions as whether the material can be grown outside of its traditional growing regions and still reflects or represents the quality of material as documented in traditional literature and/or practice or whether certain agricultural or harvest and processing practices affect the therapeutic materials. As additional information is gathered, new associations can be ascertained. The interrelated data capture allows stratification of sub-data sets to reexamine the ongoing additional data, relationships and conclusions, which increase and improve with use. With each succeeding generation of use the standardized data is refined, leading to more relevant correlations to traditional use, and physicochemical and biologic/genomic characterizations are potentially improved leading to ever increasing levels of modern scientific reproducibility and quality control.

The disclosed systems can integrate physicochemical and/or biologic/genomic data from other sources, for example, to refine a control positive or control negative. One or all of the physicochemical analyses or one or all of the biologic/genomic analyses data can be imported from another research. Preferably, the data from other sources is assigned different values depending upon the quality of the source. For example, physicochemical and/or biologic/genomic data from a specimen that that was qualitatively analyzed using the systems and methods described herein can get a high rating. Data that came from a genomic database on a traditional Chinese medicine that did not use qualitative analysis or did not perform at least two physicochemical analyses would get a low rating. Accordingly, researchers could filter out only data that does not meet a minimum criteria for identity or just do a wider search knowing some of the data mat be suspect.

The systems and methods disclosed herein are useful in a variety of applications in areas including, but not limited to, research, manufacturing and QC/QA of traditional Chinese medicines. Example applications include the development of traditional Chinese medicine with modern drug-like reproducibility, identification and authentication of botanicals, identification of adulterants, comparative evaluation of adulterants, comparative source evaluation of botanicals, comparative time studies (harvests from year to year), shelf-life studies, reference specimen development, standardization and characterization of any complex natural material, preclinical screening using traditional bioassay and/or gene expression assay, and signature gene panel development specific to the activity(ies) of the botanical materials being used.

The systems and methods are also useful in marker compound quantification. The quality and dose of an herb is a key determinant for application in health and disease. A challenge of herbal-genomics is integration of herbal systems biology to identify biomarkers that can predict the beneficial or adverse effects of herbs or herbal components. Molecular diagnostics using microarray technology can help to address herb mechanisms and safety. In addition, herb-mediated proteome (proteomics), herb-mediated metabolite production (metabolomics) and appropriate bioinformatics can further our understanding of herb-modulated homeostasis and toxicology.

The systems and methods disclosed herein will be further illustrated in the following Examples.

### EXAMPLE 1

### FEVERFEW

Feverfew has traditionally been used for treating inflammatory diseases such as arthritis and rheumatism. Feverfew does not have a centuries-old tradition associated with its use in treating migraines.

However, during the past two decades, three clinical trials using dried feverfew leaf powder yielded positive results in migraine prevention. These clinical trials are described in E.S. Johnson, N.P. Kadam, D.M. Hylands, et al., Efficacy of Feverfew as Prophylactic Treatment of Migraine, British Medical Journal (1985) 291:569-573; JJ. Murphy, S. Heptinstall & J.R. Mitchell, Randomised Double-Blind Placebo-Controlled Trial of Feverfew in Migraine Prevention, Lancet (1988) 2:189-192; and D. Palevitch, G. Earon, R. Carasso, Feverfew (Tanacelum Parthenium) as a Prophylactic Treatment for Migraine: A Double-Blind Controlled Study, Phytotherapy Research (1997) 11:508-511.

Two clinical trials using a carbon dioxide supercritical fluid extract (SFE) also yielded positive results in migraine prevention. These clinical trials are described in V. Pfaffenrath, H.C. Diener, M. Fischer, et al., The Efficacy and Safety of Tanacetum Parthenium (Feverfew) in Migraine Prophylaxis - A Double-Blind, Multicentre, Randomized Placebo-Controlled Dose-Response Study, Cephalalgia (2002) 22:523-532 and H.C. Diener, V. Pfaffenrath, J. Schnitker, M. Friede, et al., Efficacy and Safety of 6.25 mg t.i.d. Feverfew COj-Exlract (M1G-99) in Migraine Prevention - A Randomized, Double-Blind, Multicentre, Placebo-Controlled Study, Cephalalgia (2005) 25:1031-41.

Parthenolide is commonly thought to be the active component in feverfew responsible for anti-migraine activity. However, another well-designed clinical trial using a 90% ethanol extract that contained high levels of parthenolide (0.35%) produced negative results. This clinical trial was described in CJ. De Weerd, H.P.R. Bootsma & H. Hendriks, Herbal Medicines in Migraine Prevention: Randomized Double-Blind Placebo Controlled Crossover Trial of a Feverfew Preparation, Phytomedicine (1996) 3:225-230.

Feverfew plants were obtained that were genetically descended from the feverfew plants used in one of the successful positive clinical trials using powdered leaf ("the Heptinstall accession").

Seeds of feverfew varieties that were taxonomically identical to, but not genetically descended from, the feverfew plants used in the positive clinical trials {Tanacetum parthenhtm) were also obtained from various sources. These seeds were not authenticated as representing the quality of traditional Chinese medicine as documented in literature because their efficacy on migraine had not been documented.

Seeds of related species (e.g., tansy (*Tanacetum vulgare*), mugwort (Artemisia vulgaris), and German chamomile (*Matricaria recutita*) were also obtained from various sources. These seeds were not authenticated as representing the quality of traditional Chinese medicine as documented in literature because their efficacy on migraine had not been documented.

All plants were grown in two locations, Santa Barbara, California, U.S.A. and Charleston, South Carolina, U.S.A. to determine the effect of environmental influence in chemotypical expression.

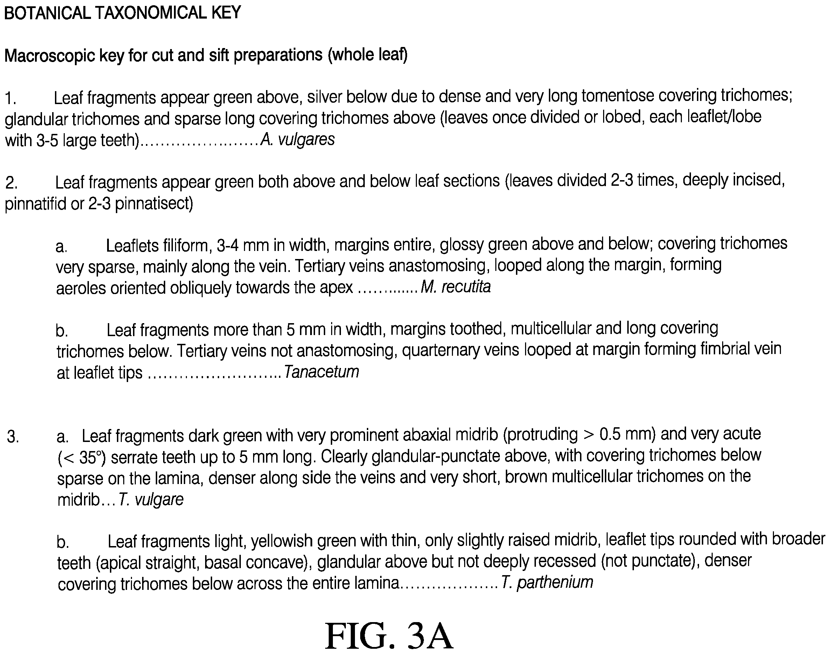

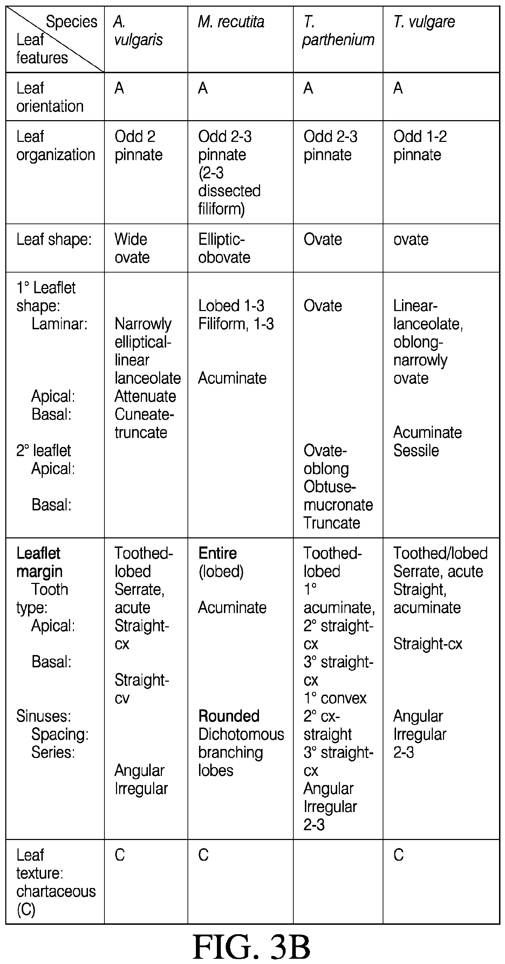

Macroscopic and microscopic characteristics of different feverfew materials (including above ones related to positive clinical trials) were compared to those of related species (tansy, mugwort, etc.). Based on their differences and similarities, identification criteria including a botanical taxonomical key and a comparative matrix were developed, as shown in FIG. 3A and FIG. 3B, respectively.

Based on the above-described published clinical trial results, it was deduced that the active anti-migraine components should be: relatively non-polar, present in a mild 90% EtOH extract of the dried leaf, present in a CO₂ extraction of dried leaf, and absent in a prolonged 90% EtOH extract of the dried leaf. Accordingly, three different methods of feverfew extraction were used for this study, as shown below in TABLE 1. The three extracts described in TABLE 1 (SRE, SFE, and DeWe) were prepared from plant biomass grown from the Heptinstall accession.

**TABLE 1 : METHODS OF FEVERFEW EXTRACTION**

| Name | Description |
|---|---|
| SFE (Control Positive) Carbon dioxide supercritical fluid extract | Conditions (400 bar, 60 °C) that yielded the broadest spectrum of extracted chemicals were used to stimulate the supercritical fluid extract (SFE) reported in the literature as yielding positive results |
| SRE (Control Positive) Standard Reference Extract | A 90% ethanol extract prepared under mild conditions (sonification for 30 minutes without evaporation, no heat, minimized exposure to oxygen) was used to stimulate the dried leaf power with all the active components |
| DeWe (Control Negative) | A simulated de Weerdt extraction procedure was used to stimulate the failed clinical trails using a high Parthenolide extract |

Because both the method of preparation and the genetic strain of SFE and SRE matched the documentation in literature which resulted in anti-migraine efficacy, the SRE and SFE represented control positives, specimens that represented the quality of traditional Chinese medicine as documented in literature. Because the method of preparation of the DeWe was previously established not to be effective in treating migraines, the DeWe represented a control negative, a specimen that did not represent the quality of traditional Chinese medicine as documented in literature.

Each of the feverfew extracts was analyzed using a plurality of physicochemical profiling techniques, namely, a combination of HPTLC, GC, UV/VIS, FTIR, HPLC, GC/MS, and others. The differences among the physicochemical fingerprints obtained from the SRE, SFE (control positive) and DeWe (control negative) extracts were compared in order to locate the components which are present in SRE and SFE but absent in DeWe.

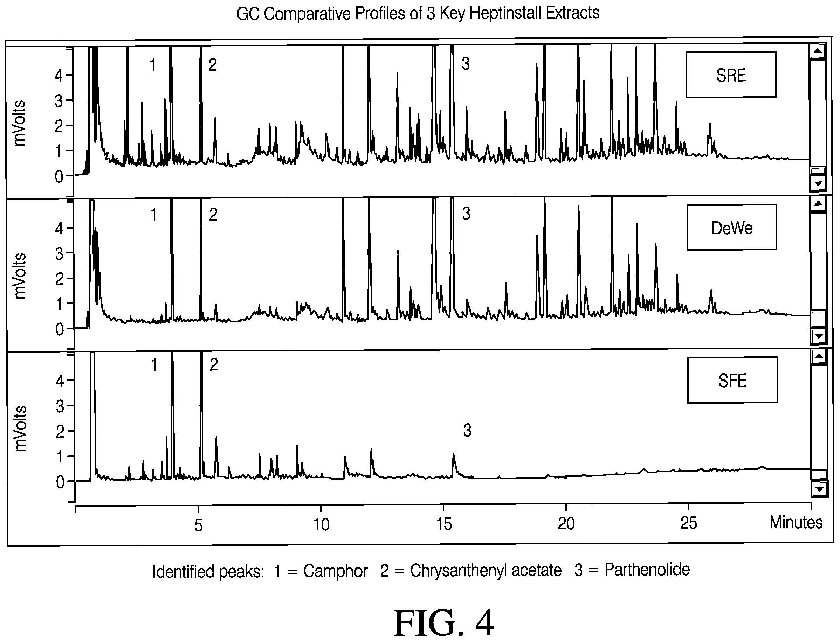

Results showed certain distinct similarities among SFE and SRE that are not present in DeWE. As shown in FIG. 4, the ratio of three components (labeled 1, 2, and 3) present in GC of the three extracts showed a consistent pattern. These components were later identified to be camphor, chrysanthenyl acetate, and parthenolide, accordingly. However, it is important to note that identification of the components is not necessary. The scientifically- reproducible systems and methods for assessing traditional Chinese medicines described herein do not require the isolation and/or identification of active constituents. The broad spectrum of components in a traditional Chinese medicine can be characterized without inquiring into the components' identities.

FIG. 4 further shows that in both the SRE and SFE, camphor and chrysanthenyl acetate predominate over parthenolide, while parthenolide predominates over the other camphor and chrysanthenyl acetate in the DeWe.

The GC analysis in FIG. 4 further exemplifies the potential for misinformation by relying on only one physicochemical analytical methodology. The inactive extract, DeWe, appears more similar to the primary active control positive extract, SRE, then it does to the other control positive extract, SFE.

FIG. 4 further shows that the previously presumed active chemical component, parthenolide, is high in the control negative, DeWe and only one of the control positives, SER, clearly indicating that parthenolide is not a singular active chemical component if at all.

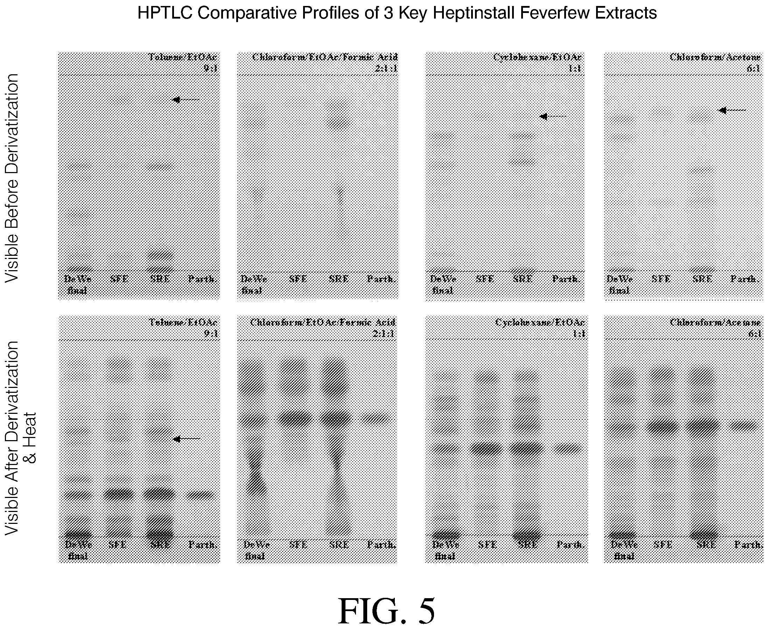

HPLC and HPTLC fingerprints for the three extracts showed other unidentified compounds consistently present in SFE and SRE but absent in DeWe. This is demonstrated in the HPTLC data shown in FIG. 5. The arrows in FIG. 5 point to commonalities between the control positives, SFE and SRE, not found in the control negative, DeWe, indicating possible compounds relevant to migraine activity. The parthenolide specimen (DeWe) on comparison to the whole plant extracts demonstrates the importance in fingerprint analysis like HPTLC, as it tends to show a range of potentially active components beyond the currently-presumed industry noπn of parthenolide as the marker standard reference sample and the active component.

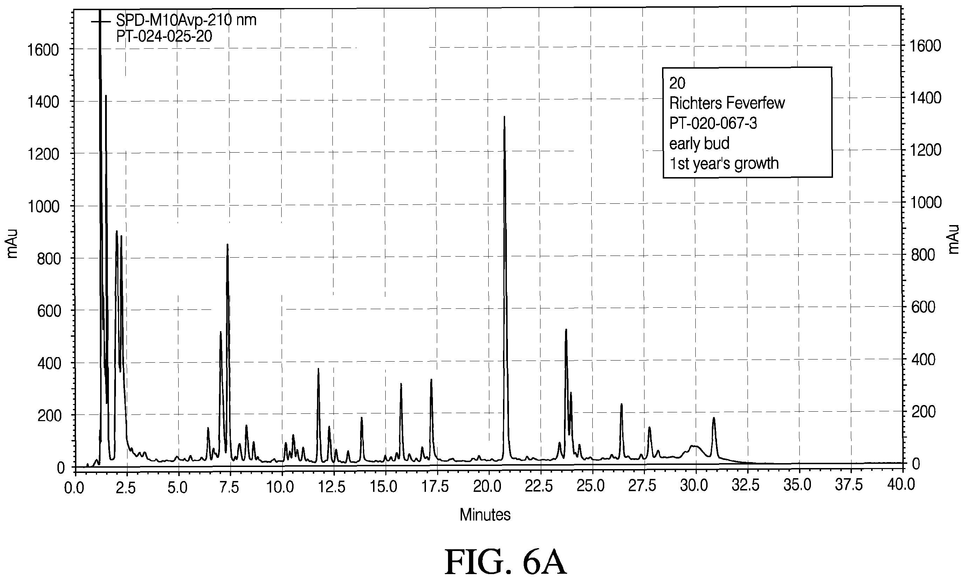

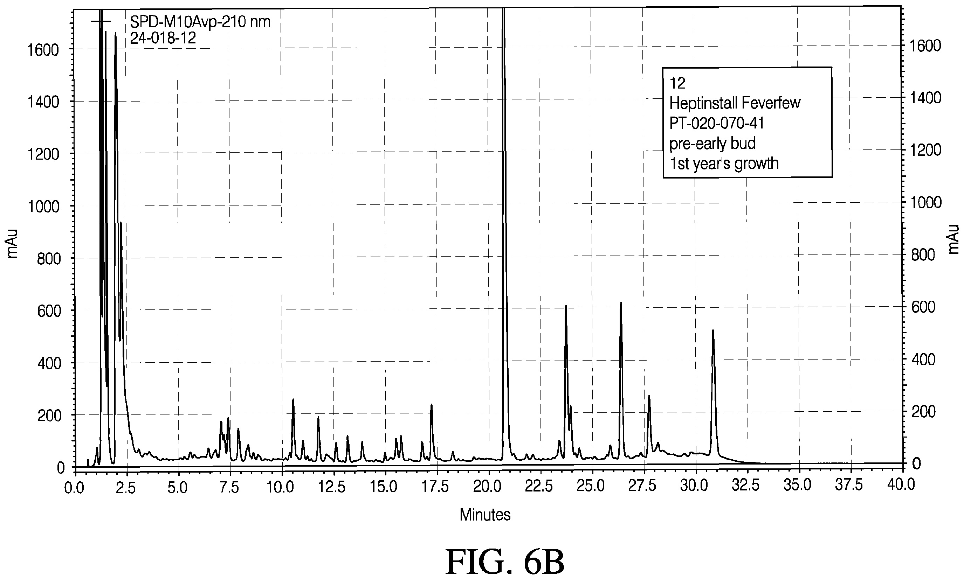

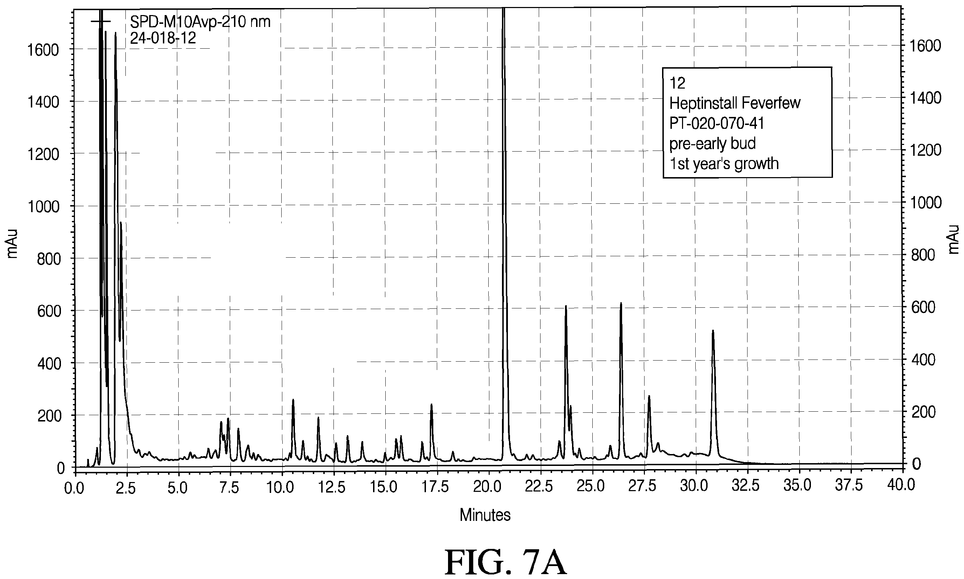

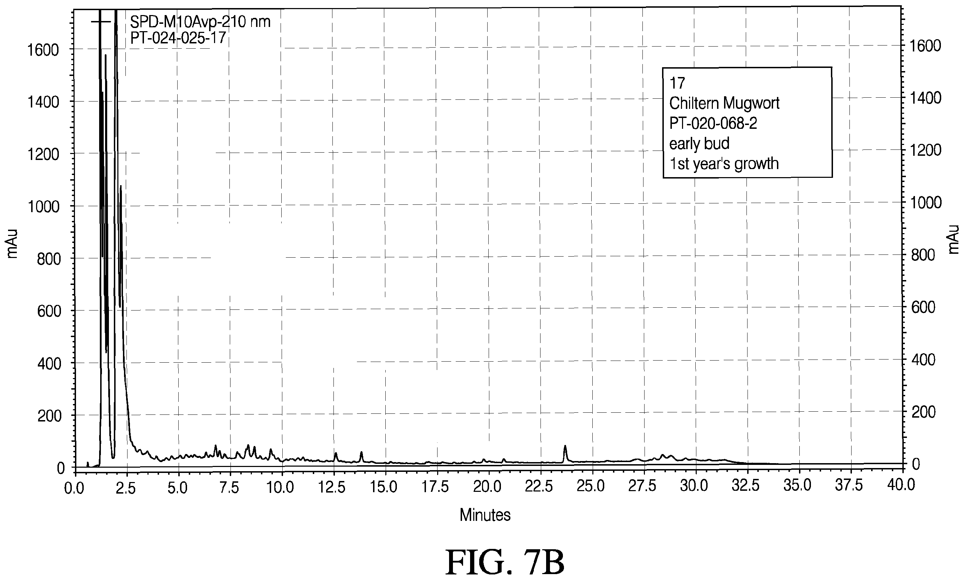

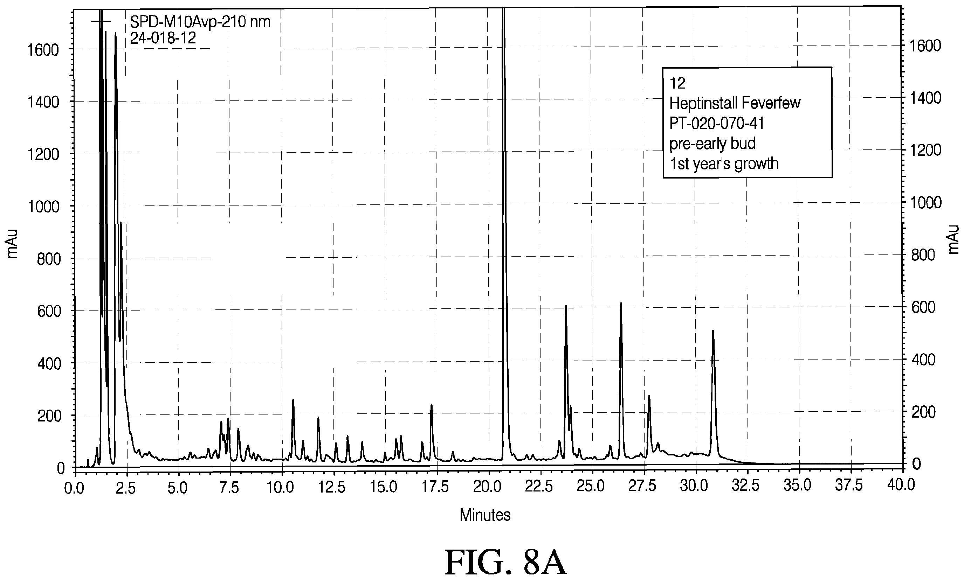

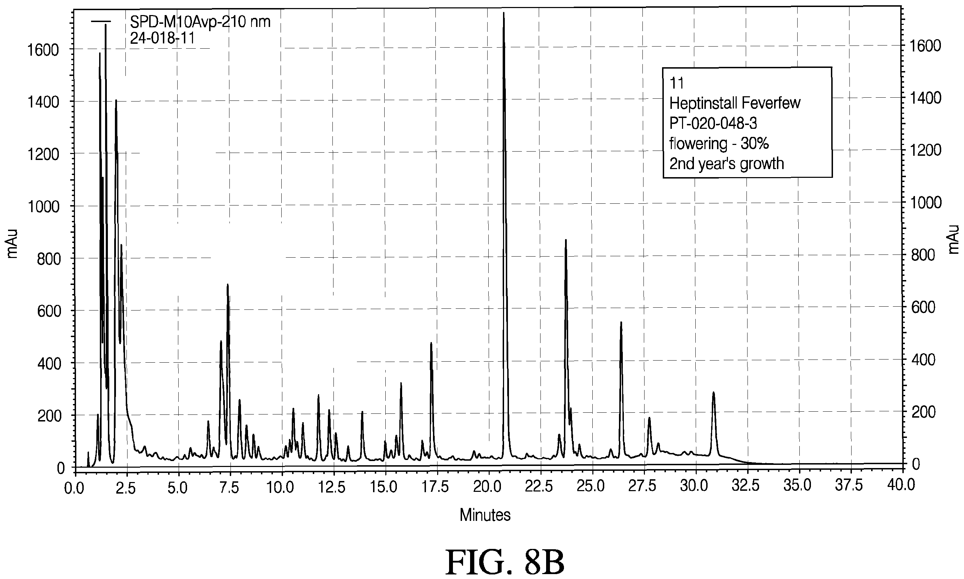

HPLC is also valuable tool for quantitatively analyzing traditional Chinese medicine specimens and contributing to a specimen's physicochemical fingerprint. HPLC data is presented for two feverview varieties, Richter's (FIG. 6A) and Heptinstall (FIG. 6B). In this comparison, HPLC was able to show some overall differences between these two feverfew varieties. As another example, HPLC data shows a significant difference between feverfew (FIG. 7A) and a different species, mugwort, *Artemisia vulgaris* (FIG. 7B). HPLC can also be used to detect a quantitative change in a group of compounds in the Heptinstal] feverfew from the first year growth (FIG. 8A) and the second year growth (FIG. 8B). The therapeutic relevance of these changes can be determined in conjunction with other quantitative techniques comparing the control positive(s), including the addition of bioassay.

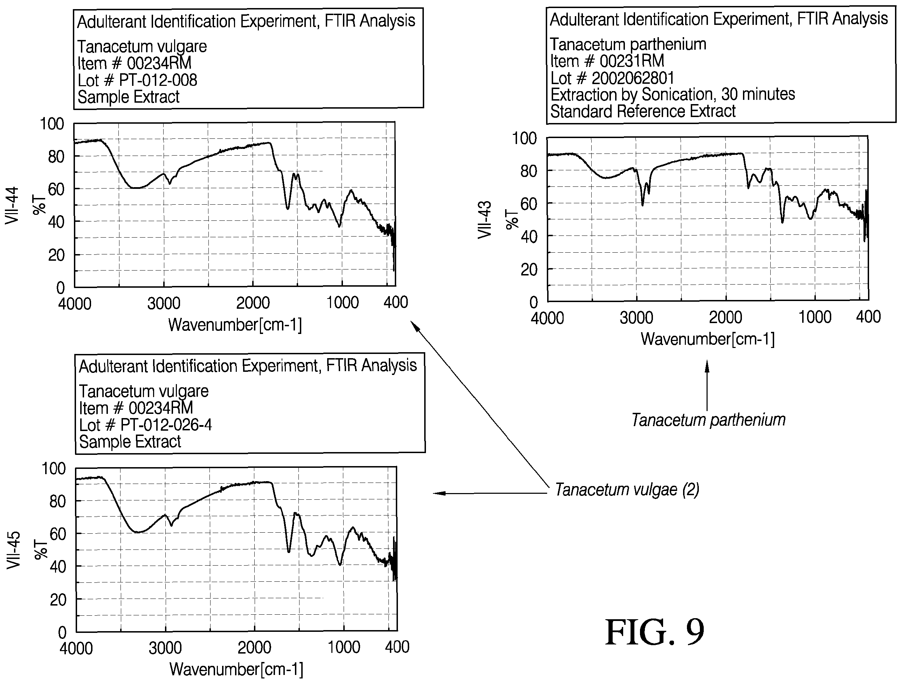

The FTIR analysis between three specimens of the same genus *Tanacetum* (FIG. 9) shows a consistent pattern between the two specimens of *Tanacetum vulgar* and a distinctly different pattern for *Tanacetum parthenium* (feverfew). FIG. 9 demonstrates the value of the broad spectrum technology of FTIR to show adulteration including incorrect species.

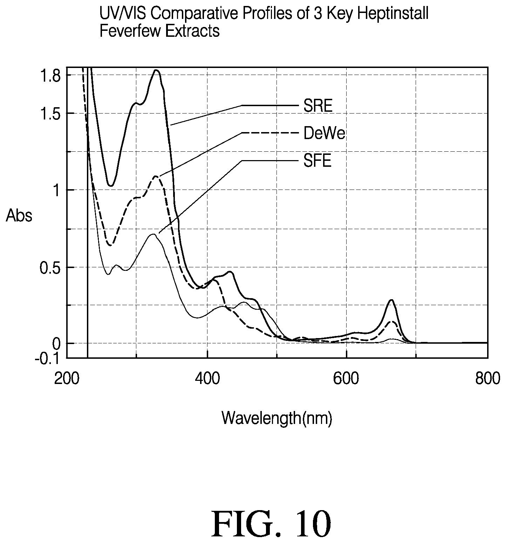

FIG. 10 shows the results of the UV/VIS analysis of the three extracts. While there are some differences apparent between between the control positives and control negative specimens, the UV/VIS shows a relatively high degree of commonality between all specimens. FIG. 10 reinforces the importance of not relying on a single physicochemical analysis when fingerprinting a traditional Chinese medicine. The systems and methods disclosed herein comprise multiple, broad-based analytical techniques for physicochemical characterization

Biologic/genomic tests were further conducted. A range of bioassays were implemented. Example results are presented from the DNA microarray, which allowed simultaneous measurement of the gene expression of the entire genome and provided a global view of gene-gene interaction network and biological processes. DNA microarray advantageously does not require a priori assumptions or knowledge of the biological system. Based on the patterns of gene expression, the genomic microarray approach can give indications of biochemical and molecular targets of herbs in the cells.

Cells were treated with solvent (control) or nodulating bacteria. To minimize sampling variation and experimental errors and to maximize the contrast between specimens, a loop design with dye swap was implemented as described in M.K. Kerr & G.A. Churchill, Experimental Design for Gene Expression Microarrays, Biostatistics (2001) 2:183-201 and M.K. Kerr & G.A. Churchill, Statistical Design and the Analysis of Gene Expression Microarray Data, Genetic Research (2001) 77:123-128. Total RNA was isolated using Agilent Total RNA Isolation Mini Kit (Agilent Technologies, Wilmington, DE). RNA specimens were labeled with Cy3 or Cy5 fluorescent dye and hybridized for 17 hours. Image processing and fluorescence intensity were interpreted and analyzed by Agilent Feature Extraction (version 8.5).

Data normalization was performed by a nonlinear LOWESS method that uses gene intensity and spatial information. Gene expression fold changes were calculated as ratio of treatment (i.e. feverfew) divided by control (solvent only). For statistical analysis, the fold change ratios were then transformed into logarithmic scale. To select the genes that were differentially expressed, we used the concept of false discovery rate (FDR). For each treatment, a modified t-test was performed for four independent experiments using the software program, Significance Analysis of Microarray (SAM). Several bioinformatics methods for data analysis were used including clustering.

The Gene Ontology database is a large collaborative public database of controlled biological vocabularies (i.e. ontologies) that describe gene products based on their functionalities in the cell. In general, over- or under-representation of GO terms can be evaluated using the Fisher Exact Probability Test. We utilized the on-line program FatiGO to produce (1) percentage and number of genes appearing in a GO category; (2) p-values from the Fisher exact test for each GO term associated with the gene.

The design of primer sequences using an online tool and the oligos both were from Integrated DNA Technologies (Coralville, IA). Our result suggested that mRNAs of GAPDH gene were not affected by treatments (data not shown) and was therefore used as the reference. Genes of interest and reference genes (i.e. GAPDH) were PCR-amplified for construction of standard reference sample curves.

Total RNA for both treatment and control groups were isolated using Absolutely RNA RT-PCR Miniprep Kit from Stratagene (La Jolla, CA). Primer concentration were optimized individually. Brilliant SYBR Green QRT-PCR Master Mix Kit (1-Step) from Stratagene was used to prepare the reaction mix. Reactions were carried out on an Mx3000 Real-time RT-PCR machine from Stratagene. Each experiment was performed in triplicate. Expression change of the gene was calculated by standard reference sample curve method and normalized to the reference gene.

Gene expression level of the feverfew treated cells were compared with controls. As shown in FIG. HA, the numbers of significant gene responses due to treatment of each extract varied. Sixteen genes responded to all three extracts, shown in detail in FIG. HB. These 16 common genes likely represent a core "immune" gene set of human macrophages for feverfew extracts. As shown in FIG. HC, expression profiling of the core immune genes suggest that genes that response to SRE and SFE are more similar than those to DeWe.

When subjecting the three extracts to genomic profiling using microarray, 198 genes responded to SFE and SRE but not to DeWe. Out of the 198 genes common between the control positives (SFE and SRE), 28 signature genes were selected which can be used as a lower cost genomic test to identify the potential anti-migraine extracts/fractions that have exhibited physicochemical profiles commensurate with anti-migraine activity. This combined multicomponent technique, biologic/genomic and physicochemical profiling, can be used to identify, characterize and standardize feverfew materials that hold the biological and chemical characteristics necessary for anti-migraine activity.

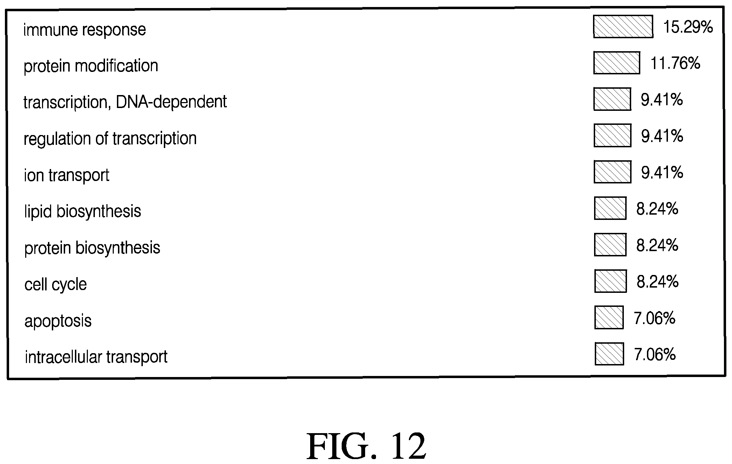

Using informatics we can also gain potential insights of mechanisms of action to identify gene response patterns to previously known physiological associations, as shown in FIG. 12. Not only are we able to gather information from prior genomic research, but also we are able to add information based on traditional use, expanding the information potential of the genomic databases for a variety of uses.

### EXAMPLE 2

### GINSENG

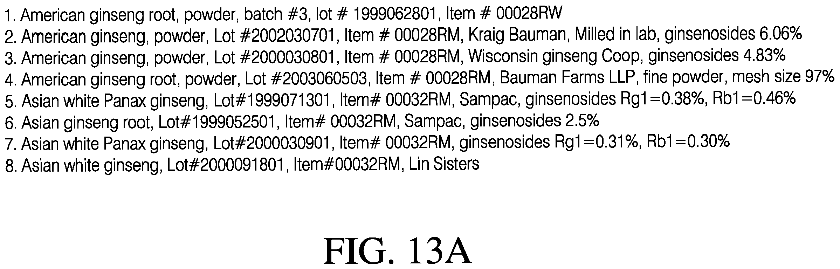

Eight specimens were prepared, four specimens using 2 g powdered *Panax quinquefolius* (American ginseng) from various sources and four specimens using 2 g powdered *Panax ginseng* (Asian ginseng), in 20 mL 90% EtOH with sonication. The sources are described in greater detail in the table shown in FIG. 13A.

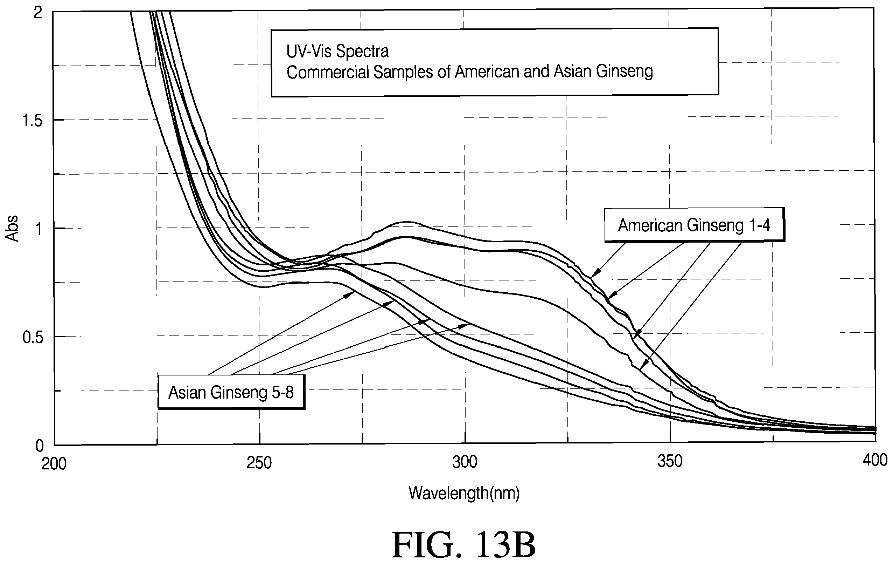

A resulting UV-VIS spectra is shown in FIG. 13B. The UV/VIS spectra shows a clear differentiation between Asian and American ginseng. The usual technique of using ginsenoside as a marker compound would not show the differentiation. Furthermore, in sharp contrast to FIG. 10 in which UV-VIS analysis showed a relatively high degree of commonality between all specimens that was not generally helpful in distinguishing between specimens, FIG. 13B shows that UV-VIS can be an effective fingerprinting tool when used in combination with other physicochemical analytical techniques.

### EXAMPLE 3

### TURMERIC

There has been much confusion regarding the common spice and traditional Chinese medicine turmeric. According to traditional practice and documentation, turmeric originally was the rhizome of *Curcuma longa* ("*rhizoma Curcumae longae*). However, over the years, materials from other plant parts and even from other species have also been used and introduced into commerce as "turmeric." The most prominent ones are: the root tuber of *Curcurma longa* ("radix *Curcumae longae* ') and other *Curcuma* spp. ("*radix Curcumae*") and the rhizome of other *Curcuma* spp. ("*rhizoma Curcumae*").

Three herbs that are recognized in traditional Chinese medicine (TCM) are known in Chinese as (1) *Jianghuang* (turmeric, the rhizome of only *Curcuma longa* or *rhizoma Curcumae longae*), (2) *Yujin* (the root of *Curcuma longa* and other *Curcuma* spp. or *radix Curcumae*), and (3) *Ezhu* (the rhizome of *Curcuma* spp. other than *Curcuma longa* or *rhizoma Curcumae*). These are tabulated below in TABLE 2 along with their plant sources.

**TABLE 2: TURMERIC AND RELATED BOTANICALS**

| Botanical | Source Plant | Plant Part |
|---|---|---|
| *Jianghuang*/Turmeric | 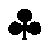 *Curcuma longa* L. | rhizome |
| *Yujin* | 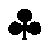 *Curcuma longa* | root tuber |
| | 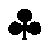 *C. wenyujin* Y.H. Chen& C. Ling | root tuber |
| | 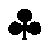 *C.kwangsiensis* S.G.Lee&C.F.Liang | root tuber |
| | 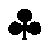 *C.phaeocaulis* Val. | root tuber |
| | 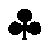 *C. aeruginosa* Roxb. | root tuber |
| | 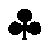 *C.aromatica* Salisb. | root tuber |
| | 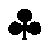 *C.zedoaria* (Christm.) Roscoe | root tuber |
| *Ezhu* | 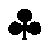 *Curcuma wenyujin* | rhizome |
| | 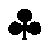 *C. kwangsiensis* | rhizome |
| | 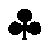 *C.phaeocaulis* | rhizome |
| | 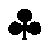 *C. aeruginosa* | rhizome |
| | 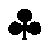 *C.aromatica* | rhizome |
| | 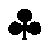 *C.zedoaria* | rhizome |

| | | |
|---|---|---|
| 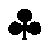 Official in the *Chinese Pharmacopoeia* 2000 | | |

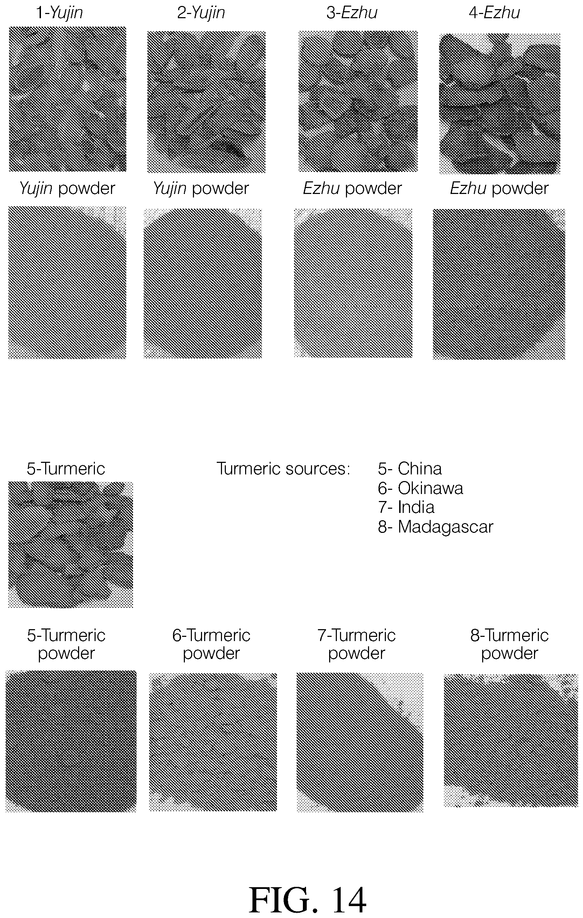

Although there may be confusion in using the rhizome of other species than *C*. *longa*, TCM clearly distinguishes the three botanicals (turmeric, *ezhu* and *yujin*) with similar but distinctly different properties and indications. The taxonomic classification system used in the West would also have characterized the root tuber of Curcuma longa and the root tuber *Curcuma zedoaria* as different botanical therapeutics. In TCM, they are both called *Yujin* (*radix Curcumae*) and share a common traditional practice and documentation. This is an additional example of the importance of correctly matching the botanical materials to the traditional literature / practice. Drawings showing the whole and powdered forms of ezhu and yujin and whole and powdered forms of turmeric from various sources are shown in FIG. 14.

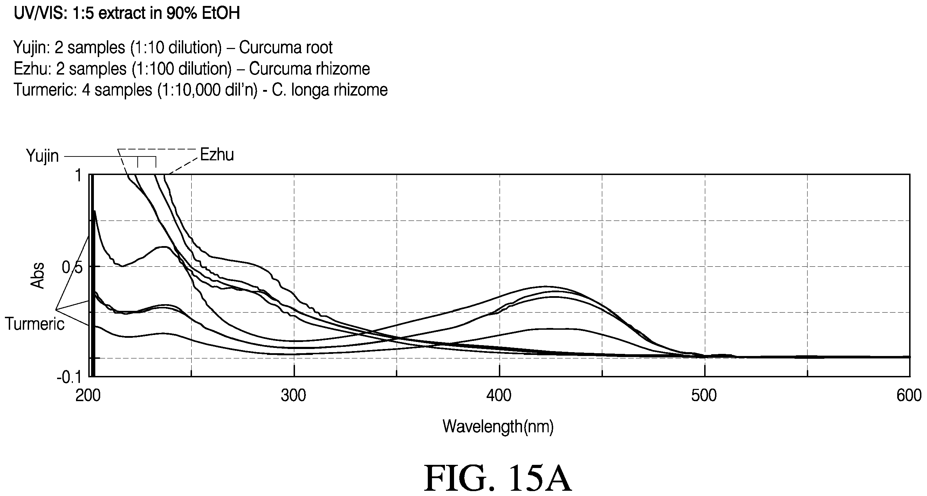

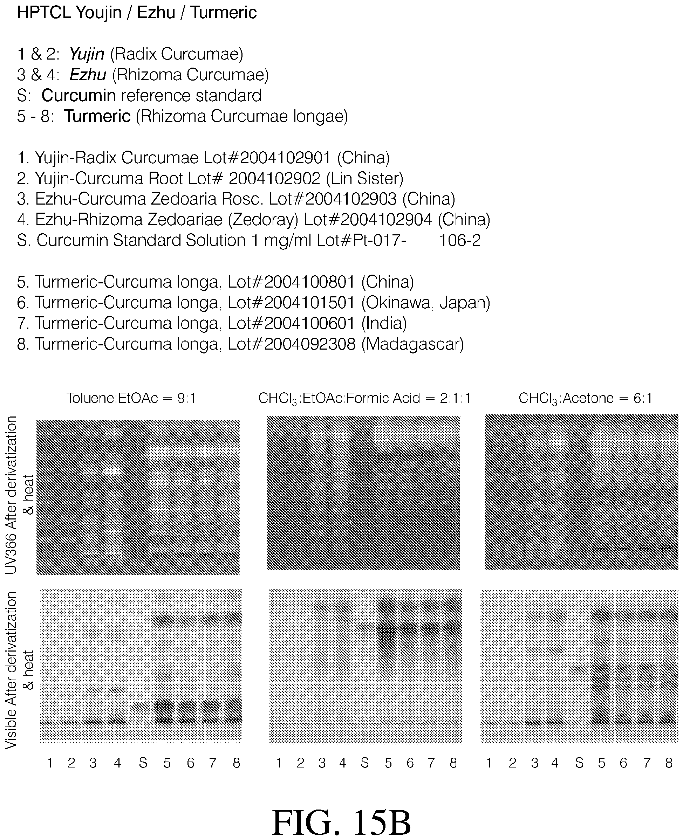

The UV/VIS and HPTLC of turmeric (botanical) vs. *yujin* and *ezhu* (traditional) are provided in FIG. ISA and FIG. 15B, respectively. In the West, *yujin*, *ezhu*, and turmeric are generally called "turmeric," and not recognized as being different. The UV-VIS and HPTLC bolster the traditional differentiation between *Yujin*, *Ezhu*, and Turmeric that is absent in the West. The HPTLC specimens in columns 5 through 8 of FIG. 15B are an example of the regional differences in the same species from different areas. These specimens show general similarity; however, some differences are captured, showing variation of material that may be found in traditional practice and current commerce.

### EXAMPLE 4

### GANODERMA

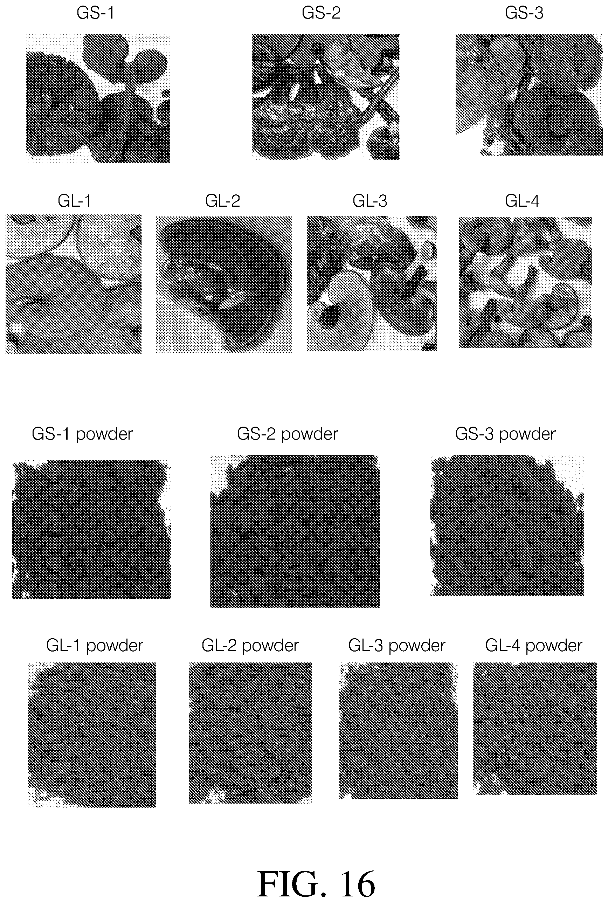

*Ganoderma lucidum* (Reishi, lingzhi) (red) and *Ganoderma sinensel, G. japonicum* (purple) were compared. As show in FIG. 16, visually the Ganoderma sinense (GS) looks darker than the *Ganoderma lucidum* (GL). However, as the GL ages, it darkens. This darkening often creates confusion with Western researchers who do not have an authenticated control positive specimen and do not know where to find this information in the traditional literature. The researchers' confusion can call into doubt which material may have been used in a research project.

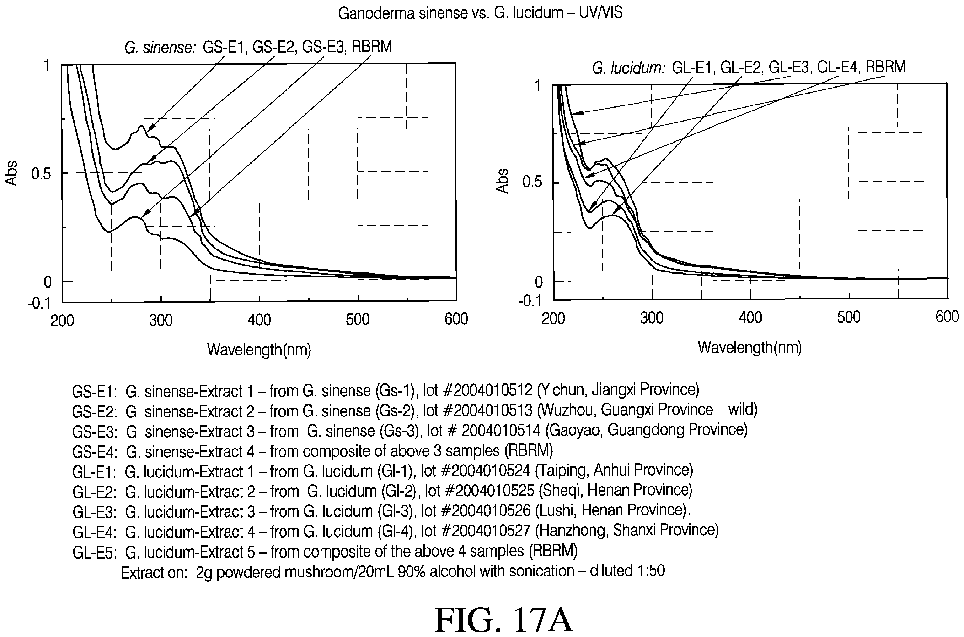

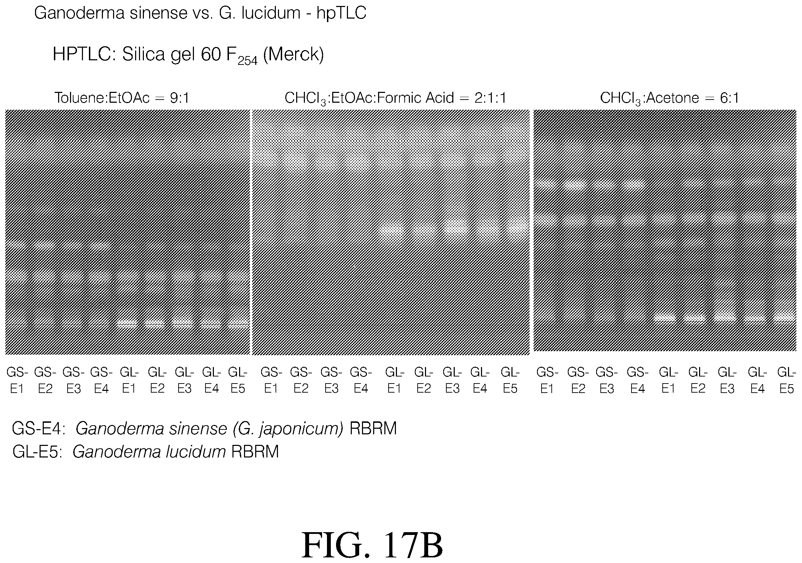

The resulting UV-VIS and HPTLC analyses are shown in FIG. 17A and FIG. 17B, respectively. One specimen labeled RBRM represents a standard reference sample created from blended control positives. The standard reference sample includes the range of variation inherent in the herb as it has been traditionally used and documented for centuries, and this variation is captured in the UV VIS and in the HPTLC (columns GS E4 and GL E5).

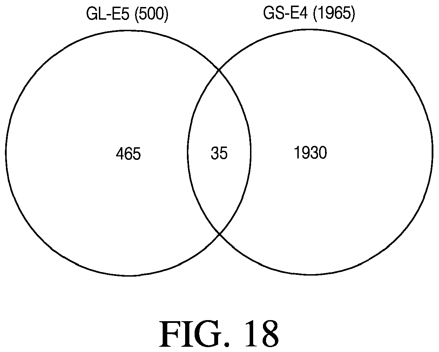

The Venn diagram shown in FIG. 18 demonstrates the number of differentially expressed genes activated by GL-E5 and GS-E4. FIG. 18 shows 35 genes expressed in common. GL and GS are often used interchangeably or confused in the west. Although in traditional use they have some similar therapeutic actions, they are considered distinct. The UVVIS, HPTLC and biologic/genomic profiles all confirm the ability of appropriate scientific analytical techniques to verify traditional knowledge and practice.

## Claims

1. A method of defining a standard reference sample for a whole traditional Chinese medicine, the method comprising:
a) obtaining at least two specimens of the whole traditional Chinese medicine consisting of *Panax quinquefolius, Panax ginseng, Curcuma spp., Ganoderma lucidum, Ganoderma sinense* that have been authenticated as representing a positive control by qualitative profiling using an organoleptic examination;
b) quantitatively profiling each of the at least two specimens that are authenticated as the positive control in step a) using High Performance Thin Layer Chromatography in combination of High-Performance Liquid Chromatography, Fourier Transform Infrared Spectroscopy and/or Gas-Liquid Chromatography and quantitatively profiling at least one of the at least two speciments using at least one biologic/genomic analysis selected from group consisting of proteomic assay, clinical trial, and DNA microarray to confirm positive authenticity of the specimens;
c) blending at least two speciments that are authenticated as the positive control in step a) and confirmed in the step b) to form the standard reference sample;
d) and creating a quantitative profile for the standard reference sample using High Performance Thin Layer Chromatography in combination of High-Performance Liquid Chromatography, Fourier Transform Infrared Spectroscopy and/or Gas-Liquid Chromatography and quantitatively profiling of the standard reference sample using at least one biologic/genomic analysis selected from group consisting of proteomic assay, clinical trial, and DNA microarray; wherein the quantitative profile for the standard reference sample defines the standard reference sample.

2. A method of evaluating a test specimen of a whole traditional Chinese medicine, the method comprising:
a) creating a quantitative profile for the test specimen using at least two physicochemical analyses selected from the group consisting of Mass Spectrometry, High Performance Thin Layer Chromatography, Fourier Transform Infrared Spectroscopy, Ultraviolet-Visible Spectroscopy, Thin Layer Chromatography, Gas-Liquid Chromatography, High-Performance Liquid Chromatography, Liquid Chromatography-Mass Spectrometry, and Gas Chromatography-Mass Spectrometry and creating the quantitative profile for the test specimen using at least one biologic/genomic analysis selected from the group consisting of proteomic assay, clinical trial and DNA microarray or combination thereof;
b) providing a standard reference sample defined by the method according to claim 1;
c) comparing the quantitative profile for the standard reference sample to the quantitative profile for the test specimen mathematicaly or visually;
d) evaluation being reached when the variation between the test specimen and the standard reference sample is 25% or less, preferably 10% or less or the most preferably below 5%.

3. The method of Claim 2, further comprising authenticating the test specimen by qualitative profiling.

## Patentansprüche

1. Verfahren zur Bestimmung der Definition einer standardmäßigen Referenzprobe für eine ganze traditionelle chinesische Medizin, wobei, wobei das Verfahren umfasst:
a) Entnahme von wenigstens zwei Proben der ganzen Traditionellen chinesischen Medizin, bestehend aus *Panax quinquefolius, Panax ginseng, Curcuma spp., Ganoderma lucidum, Ganoderma sinense,* die durch eine mithilfe einer organoleptischen Untersuchung durchgeführte qualitative Profilierung als derartige Proben authentifiziert wurden, welche eine positive Kontrollprobe darstellen;
b) quantitative Profilierung jeder der wenigstens zwei Proben, die in dem Schritt a) als positive Kontrollproben authentifiziert wurden, mithilfe der Hochleistungs-Dünnschichtchromatographie in Kombination mit der Hochleistungs-Flüssigkeitschromatographie, der Fourier-Transformations-Infrarotspektroskopie und/oder der Gas-Flüssigkeitschromatographie sowie quantitative Profilierung wenigstens einer der wenigstens zwei Proben mithilfe wenigstens einer biologischen / genomischen Analyse, die aus der Gruppe ausgewählt wird, die proteomische Untersuchung, klinische Studie und DNA-Microarray-Technologie zur Bestätigung der positiven Authentizität der Proben umfasst;
c) Vermischung von wenigstens zwei Proben, die in dem Schritt a) als positive Kontrollproben authentifiziert wurden und bei den es in dem Schritt b) bestätigt wurde, sodass sie die standardmäßige Referenzprobe bilden;
d) und Aufstellung eines quantitativen Profils für die standardmäßige Referenzprobe mithilfe der Hochleistungs-Dünnschichtchromatographie in Kombination mit der Hochleistungs-Flüssigkeitschromatographie, der Fourier-Transformations-Infrarotspektroskopie und/oder der Gas-Flüssigkeitschromatographie sowie quantitative Profilierung der standardmäßigen Referenzprobe mithilfe wenigstens einer biologischen / genomischen Analyse, die aus der Gruppe ausgewählt wird, die proteomische Untersuchung, klinische Studie und DNA-Microarray-Technologie umfasst, wobei das derart erhaltene Profil der standardmäßigen Referenzprobe die standardmäßige Referenzprobe definiert.

2. Verfahren zur Bewertung einer Versuchsprobe einer ganzen traditionellen chinesischen Medizin, wobei das Verfahren umfasst:
a) Aufstellung eines quantitativen Profils für die Versuchsprobe mithilfe wenigstens zwei physikochemischen Analysen, die aus der Gruppe ausgewählt werden, die Massenspektroskopie, Hochleistungs-Dünnschichtchromatographie, Fourier-Transformations-Infrarotspektroskopie, ultraviolett-sichtbare Spektroskopie, Dünnschichtchromatographie, Gas- und Flüssigkeitschromatographie, Flüssigchromatographie mit Massenspektrometrie-Kopplung und Gaschromatographie mit Massenspektrometrie-Kopplung umfasst, und Aufstellung eines quantitativen Profils für die Versuchsprobe mithilfe wenigstens einer biologischen / genomischen Analyse, die aus der Gruppe ausgewählt wird, die proteomische Untersuchung, klinische Studie und DNA-Microarray-Technologie oder deren Kombination umfasst;
b) Bereitstellung einer in dem Verfahren nach Anspruch 1 definierten standardmäßigen Referenzprobe;
c) mathematische oder visuelle Vergleichung des quantitativen Profils der standardmäßigen Referenzprobe mit dem quantitativen Profil der Versuchsprobe;
d) wobei die abschließende Bewertung dann erreicht worden ist, wenn die Abwandlung zwischen der Versuchsprobe und der standardmäßigen Referenzprobe 25% oder weniger, vorzugsweise 10% oder weniger oder am meisten bevorzugt weniger als 5% beträgt.

3. Verfahren nach Anspruch 2, wobei das Verfahren ferner die Authentifizierung der Versuchsprobe durch die qualitative Profilierung umfasst.

## Revendications

1. Un procédé de définition d'un échantillon de référence standard pour toute la médecine chinoise traditionnelle, le procédé comprenant :
a) fourniture d'au moins deux spécimens de toute la médecine chinoise traditionnelle consistant en *Panax quinquefolius, Panax ginseng, Curcuma spp., Ganoderma lucidum, Ganoderma sinense* qui ont été authentifiés comme représentant un contrôle positif par profilage qualitatif en utilisant un examen organoleptique;
b) profilage quantitatif de chacun d'au moins deux spécimens authentifiés comme contrôle positif dans l'étape a) en utilisant une chromatographie sur couche mince à haute performance en combinaison avec chromatographie liquide à haute performance, spectroscopie infrarouge à transformée de Fourier et / ou chromatographie gaz-liquide et profilage quantitatif d'au moins un des au moins deux spécimens en utilisant au moins une analyse biologique / génomique choisie dans le groupe consistant en un essai protéomique, un essai clinique et une micropuce à ADN pour confirmer l'authenticité positive des spécimens ;
c) mélange d'au moins deux specimens authentifiés en tant que contrôle positif à l'étape a) et confirmés à l'étape b) pour former l'échantillon de référence standard ;
d) et création d'un profil quantitatif pour l'échantillon de référence standard par chromatographie sur couche mince à haute performance en combinaison avec chromatographie liquide à haute performance, spectroscopie infrarouge à transformée de Fourier et / ou chromatographie gaz-liquide et profilage quantitatif de l'échantillon de référence standard en utilisant au moins une analyse biologique / génomique choisie dans le groupe constitué d'un essai protéomique, d'un essai clinique et d'une micropuce à ADN; où le profil quantitatif pour l'échantillon de référence standard définit l'échantillon de référence standard.

2. Un procédé d'évaluation d'un spécimen d'essai de toute la médecine chinoise traditionnelle, le procédé comprenant :
a) création d'un profil quantitatif pour l'échantillon d'essai en utilisant au moins deux analyses physicochimiques choisies dans le groupe consistant en spectrométrie de masse, chromatographie sur couche mince à haute performance, spectroscopie infrarouge à transformée de Fourier, spectroscopie ultraviolet-visible, chromatographie sur couche mince, chromatographie gaz-liquide, chromatographie en phase liquide à haute performance, chromatographie en phase liquide-spectrométrie de masse et chromatographie en phase gazeuse-spectrométrie de masse, et création du profil quantitatif du spécimen d'essai en utilisant au moins une analyse biologique / génomique choisie dans le groupe comprenant analyse protéomique, essai clinique et micropuce à ADN ou une combinaison de ceux-ci ;
b) fourniture d'un échantillon de référence standard défini par le procédé selon la revendication 1;
c) comparaison du profil quantitatif pour l'échantillon de référence standard au profil quantitatif pour le spécimen d'essai mathématiquement ou visuellement ;
d) l'évaluation étant atteinte lorsque la variation entre le spécimen d'essai et l'échantillon de référence standard est 25% ou moins, de préférence 10% ou moins ou le plus préférablement moins de 5%.

3. Le procédé selon la revendication 2, comprenant en outre l'authentification du spécimen d'essai par profilage qualitatif.
